# EUROPEAN PATENT APPLICATION

(11) **EP 1 595 538 A2**
(43) Date of publication of application: **16.11.2005**
(21) Application number: 05017380.6
(22) Date of filing: 06.11.2002
(51) Int. Cl.: A61K 31/18, A61K 9/20, A61K 9/28

(54) **Modified release tamsulosin tablets**

(30) Priority: 07.11.2001 US 331055 P
(62) Divisional of application: 02780143.0
(71) Applicant: Synthon B.V., 6545 CM Nijmegen (NL)
(72) Inventor: The designation of the inventor has not yet been filed
(74) Representative: Duxbury, Stephen

(57) **Abstract**

The invention relates to a pharmaceutical tablet comprising a tablet matrix having dispersed therein 0.1 to 10 mg of tamsulosin or a pharmaceutically acceptable salt thereof, and optionally having an enteric coating over said matrix, wherein said tablet is a modified release tablet and has a dissolution profile such that in each of the media SIF, FaSSIF, and FeSSIF, said tablet releases not more than 60% of said tamsulosin at 2 hours elapsed time in USP 2 apparatus using 500 ml of said media at 50-100 rpm paddle speed, to a monolithic pharmaceutical tablet, comprising 0.1 to 10 mg of tamsulosin or a pharmaceutically acceptable salt thereof, 10 wt% - 90 wt% hydroxypropyl methylcellulose, and a total tablet weight of 10 to 300 mg, to a unit dosage form for treating or ameliorating the conditions of benign prostatic hyperplasia comprising an effective amount of one or more tablets as mentioned above, and to a method for treating the symptoms of benign prostatic hyperplasia, which comprises administering to a patient in need thereof one or more tablets as mentioned above.

## Description

This application claims the benefit of priority under 35 U.S.C.§ 119(e) from prior U.S. provisional application 60/331,055, filed November 7, 2001, the entire contents of which are incorporated herein by reference.

### BACKGROUND OF THE INVENTION

The present invention relates to modified release tamsulosin tablets that exhibit little or no food effect and to unit dosage forms made therefrom.

Tamsulosin is the common name for 5-[2-[[2-(2-ethoxyphenoxy)ethyl]amino] propyl]-2-methoxy-benzenesulfonamide of the formula (1). 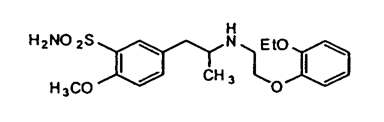 It is disclosed in EP 34432 and US 4731478 as a pharmaceutically active substance having alpha-adrenergic blocking activity that is useful for treatment of cardiac insufficiencies and benign prostatic hyperplasia.

(R)-tamsulosin hydrochloride is marketed under various tradenames, including FLOMAX® (Boehringer Ingelheim) in the U.S., HARNAL® (Yamanouchi) in Japan and OMNIC® (Yamanouchi) in Europe, for treatment of symptoms of benign prostatic hyperplasia (also known as BPH) such as urinary volume and frequency problems. The approved drug products include a capsule dosage form for oral administration that comprises 0.4 mg of the tamsulosin hydrochloride. The capsule provides controlled release of the tamsulosin and is a once daily dosage form, although two capsules can be used if needed; i.e. a maximum single daily administration of 0.8 mg.

The controlled or modified release commercialized capsule form suffers from a drawback in that it exhibits a food effect. A food effect refers to the difference in bioabsorption or bioavailability of a drug arising from administration to a fasting patient (an empty stomach) versus a fed patient (food in the stomach). For the commercial capsule, the food effect is rather pronounced. For example, it is reported in the labeling information for FLOMAX® that under fasted conditions the Tmax is 4-5 hours, but the Tmax under fed conditions is 6-7 hours. Taking the capsules under fasted conditions results in a 30% increase in bioavailability (AUC) and 40% to 70% increase in peak concentrations (Cmax) compared to fed conditions. Thus, when taken after a meal, the tamsulosin achieves a lower maximum blood plasma concentration; and this peak is achieved later in time. Accordingly, administering after a meal provides a flatter and more controlled release blood plasma profile in comparison to administering under fasting conditions, albeit with a loss in bioavailability.

The commercial capsule labeling instructs the administration to occur in a fed state: after a meal (Japan), after breakfast (Europe) and 30 minutes after the same meal each day (U.S.). It is believed that this dosing is recommended because it provides more consistent results and fewer side effects. Indeed, even though the absorption of the tamsulosin is better under fasted conditions (90+%) then fed conditions, the approved use directs that the tamsulosin capsule be administered under fed conditions.

The commercial capsule form of tamsulosin is believed to correspond to U.S. 4,772,475 (EP 194838, EP 533297). U.S. 4,772,475 discloses controlled-release pharmaceutical dosage forms comprising multiple granulate units containing tamsulosin, microcrystalline cellulose and a release control agent. The granulate gradually releases tamsulosin from the granulate matrix. No discussion occurs surrounding the food effect issue.

Because of the food effect in the commercial capsule tamsulosin product, a patient that takes the capsule while fasting (without a meal) is potentially more likely to experience undesirable side-effects such as dizziness, rhinitis, and/or abnormal ejaculation. It would be beneficial to make a tamsulosin pharmaceutical dosage form that exhibits reduced, little, or even no food effect. In this way, the dosage form would be safer; i.e. even if taken under fasted conditions, the risk of side effects is lessened. Although the food effect of the commercial tamsulosin capsule is well documented, no solution to the food effect problem has been provided thus far.

### SUMMARY OF THE INVENTION

It has now been discovered that a modified release tamsulosin tablet can be formed that has reduced food effect. Accordingly one aspect of the invention relates to a pharmaceutical tablet comprising a tablet matrix having dispersed therein 0.1 to 10 mg of tamsulosin or a pharmaceutically acceptable salt thereof, and optionally having an enteric coating over the matrix. The tablet is a modified release tablet and exhibits a dissolution profile such that in each of the media SIF, FaSSIF, and FeSSIF, each hereinafter defined, the tablet releases not more than 60% of the tamsulosin at 2 hours elapsed time in USP 2 apparatus using 500 ml of the media at 50-100 rpm paddle speed. Preferably the tablet releases at least 20% of the tamsulosin by the 2 hour mark, again in each of the three media. The media serve to model *in vitro* the intestinal conditions encountered *in vivo,* with FaSSIF corresponding to a fasting state and FeSSIF corresponding to a fed state. By having less than 60% of the tamsulosin released at 2 hours under each of the simulated conditions, the tablet demonstrates that neither fed nor fasting conditions are likely to reduce the modified release nature of the drug product.

Another aspect of the invention relates to a monolithic pharmaceutical tablet, comprising 0.1 to 10 mg of tamsulosin or a pharmaceutically acceptable salt thereof, 10 wt% - 90 wt% hydroxypropyl methylcellulose, and a total tablet weight of 10 to 300 mg. The tablet preferably exhibits reduced food effect and more preferably meets the dissolution profile requirements described above.

In each of these aspects, the tablets can be adminisered per se, optionally with an enteric coating, but preferably without an enteric coating, or one or more tablets can be encapsulated and administered as one or more capsules. A further aspect of the invention relates to a method for treating the symptoms of benign prostatic hyperplasia, which comprises administering to a patient in need thereof one or more of the above tablets.

### BRIEF DESCRIPTION OF THE DRAWINGS

FIG. 1 is the release curves of tamsulosin capsules manufactured by Yamanouchi Europe in the four described media.
FIG.2 is the release curves of tamsulosin tablets (batch G) in the four described media.
FIG.3 is the release curves of tamsulosin tablets (batch H) in the four described media.
FIG.4 is the release curves of tamsulosin enteric coated tablets (batch G) in the four described media.
FIG.5 is the release curves of tamsulosin enteric coated tablets (batch H) in the four described media.
FIG.6 is the release curves of tamsulosin uncoated and coated tablets (batch N) in selected media.

### DETAILED DESCRIPTION OF THE INVENTION

The present invention relates to modified release tamsulosin tablets and to capsules containing the same. "Modified release" is used herein in a broad sense and means any dosage form that is not an immediate release dosage form; i.e., not a dosage form that releases in a dissolution test at least 75% of the tamsulosin within the first 30 minutes in a standard dissolution test (i.e. USP apparatus 2, paddles at 50 rpm, with 500 ml SGF at 37°C). The tablets exhibit a reduced food effect in comparison to the commercially available tamsulosin capsules. Unlike these capsules, the present invention is based on the discovery that tamsulosin or its pharmaceutically acceptable salt can be formulated into a tablet having controllable food effect properties.

The tamsulosin tablets preferably exhibit a dissolution profile such that in each of SIF, FaSSIF, and FeSSIF, no more than 60%, preferably 20% to 60%, of the tamsulosin is released at 2 hours. The dissolution test is run with paddles at 50-100 rpm, preferably at 100 rpm, in USP apparatus 2 using 500 ml of the media in which the test is being conducted. In certain embodiments, especially those involving uncoated tablets, the dissolution profile further includes releasing no more than 60%, preferably 20% to 60%, of the tamsulosin in 500 ml of SGF at 2 hours is released in USP apparatus 2, paddles at 50-100 rpm, preferably at 100 rpm. In all dissolution tests for determining the release profile for purposes of the present invention, the media is at a temperature of 37 °C. 500 ml of media is used, assuming one tablet is placed in the apparatus, because it is believed to provide more accurate modeling/prediction of the *in vivo* result.

The dissolution mediums, for purposes of this invention, are defined as follows:

| SGF (USP Simulated Gastric Fluid without pepsin) composition: | |
|---|---|
| HCl | qs pH 1.2 |
| NaCl | 0.2 % |
| water | qs 1000 ml |

| SIF (USP Simulated Intestinal Fluid without pancreatin) composition: | |
|---|---|
| KH₂PO₄ | 6.8 g |
| NaOH | qs pH 6.8 |
| water | qs 1000 ml |

| FeSSIF (Simulated Intestinal Fluid, Fed State) Composition: | |
|---|---|
| Acetic acid | 0.144M |
| NaOH | qs pH 5 |
| Na Taurocholate | 15 mM |
| Lecithin | 4 mM |
| KCl | 0.19 M |
| distilled water | qs 1000 ml |
| pH=5 | |
| osmolarity=485-535 mOsm | |
| buffer capacity= 75±2 mEQ/L/pH | |

| FaSSIF (Simulated Intestinal Fluid, Fasting State) Composition: | |
|---|---|
| KH₂PO₄ | 0.029 M |
| NaOH | qs pH 6.8 |
| Na Taurocholate | 5 mM |
| Lecithin | 1.5 mM |
| KCl | 0.22 M |
| distilled water | qs 1000 ml |
| pH=6.8 | |
| osmolarity=280-310 mOsm | |
| buffer capacity= 10±2 mEQ/L/pH | |

SGF represents a standard stomach condition. SIF represents a standard intestinal condition. FeSSIF is tailored to better represent the fed state while FaSSIF is tailored to better represent the fasting state. Note that not only are the pH different, but equally important, the osmolarity are also different. FaSSIF and FeSSIF media have been generally used to describe *in vitro - in vivo* correlations for immediate release lipophilic, poorly water-soluble drugs (i.e. ketoconazole, danazol, atovaquone, troglitazone, mefenamic acid) but none of the previous studies suggest the application to a modified release low dose composition, and soluble drug such as tamsulosin HCl (volume of any aqueous media needed to dissolve until 10 mg is not more than 500 ml.), neither to a modified-release formulation (including controlled, extended or delayed release).

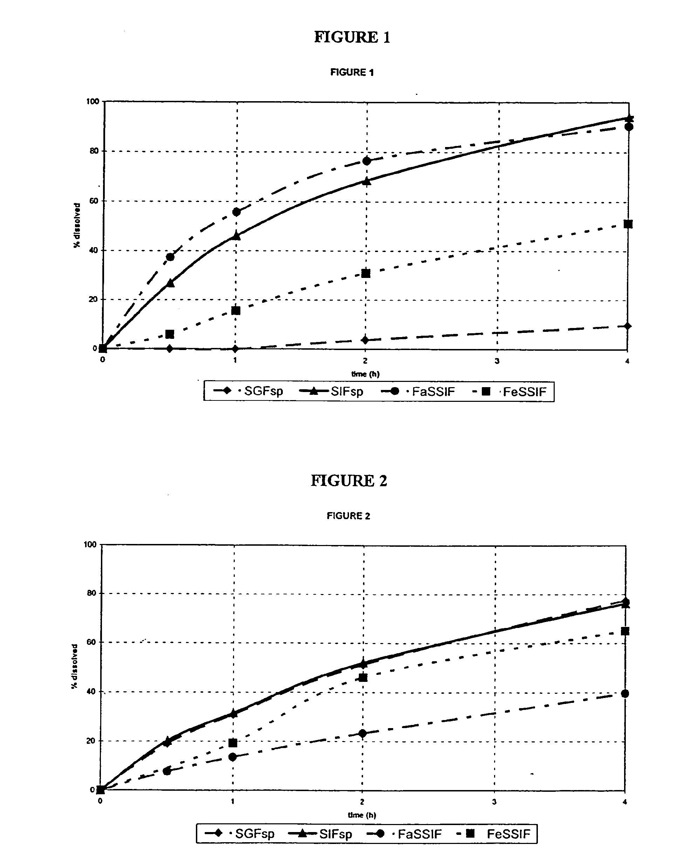

As shown in the subsequent reference example, the commercial capsules manufactured by Yamanouchi Europe, display the following release of tamsulosin at 2 hours elapsed time: more than 60% release in SIF, more than 75% release in FaSSIF while less than 40% is released in FeSSIF and less than 20% is released in SGF (see Figure 1). This divergence of results in the four media, being too fast at pH 6.8 and too slow (delayed release) at pH 1.2, indicates a possible reason for variability in plasma concentration of tamsulosin as indicated before; e.g., because of different gastric emptying and/or changing of gastrointestinal pH. Of course this more rapid release in FaSSIF corresponds well with the *in vivo* observations of a quicker Tmax and a higher Cmax in a fasting state than in a fed state. Accordingly, preferred tablets of the present invention which exhibit not more than 60% tamsulosin release in each of SIF, FaSSIF, and FeSSIF, preferably also in SGF, have a better food effect, i.e., less difference between fed and fasted conditions, than the commercial capsules.

Preferably the tamsulosin tablets release 20% to 60% of the tamsulosin during the first 2 hours of the dissolution test in each of SIF, FaSSIF, and FeSSIF. More preferably, the amount of tamsulosin released at 2 hours in FeSSIF is at least 40%, more preferably at least 50%, still more preferably at least 60% of the amount of tamsulosin released at 2 hours in FaSSIF, under the same paddle speed conditions, preferably at 100 rpm. The tablet is preferably a once daily dose tablet, which exhibits a dissolution profile in SIF within the following ranges:
<40 % in 30 minutes
20-60 % in 2 hours
>75 % in 6 hours
using USP apparatus 2, paddles at 100 rpm. More preferably the tablet also exhibits a profile within the above ranges in at least one of, and most preferably both of, FaSSIF and FeSSIF. In some embodiments, the tablet also exhibits a profile within the above ranges in SGF. It should be understood that in meeting the above ranges the dissolution profile in each media need not be identical to each other, although such is contemplated as an embodiment of the invention.

For clarity, the amount or percentage of tamsulosin released at a stated time refers to the cumulative total amount of tamsulosin released from the tablet from the start of the dissolution test up to the stated time. The amount released is determined as the average of the results from six trials; e.g. six tablets, for each media or condition. While apparatus 2 and various conditions have been specified, such is not meant to imply that the same or similar release profiles can not be obtained using different apparatus, such as USP apparatus 1 (basket), or different conditions, such as more or less media, etc. Rather, the above defined apparatus and conditions serve as a convenient way to characterize the inherent properties of the tablets of the invention.

That improved food effect could be obtained is surprising given the performance of the commercial capsule product. In general, the degree of food effect is governed by the type and solubility of the active, the amount/concentration of active, the type and concentration of polymer, and the total mass of the composition. That a modified release low dose tamsulosin tablet having a polymeric matrix could be formulated with reduced food effect, especially without a coating, is unexpected.

The tamsulosin present in the tablet is normally the (R)-enantiomer of tamsulosin but the (S)-enantiomer as well as mixtures of the two in various proportions including equimolar or racemic mixtures are also included within the meaning of tamsulosin or a pharmaceutically acceptable salt thereof. Examples of useful tamsulosin pharmaceutically acceptable salts include tamsulosin hydrochloride, tamsulosin hydrobromide, tamsulosin methane sulfonate, tamsulosin tosylate, tamsulosin besylate, tamsulosin acetate, tamsulosin maleate, tamsulosin tartrate, and tamsulosin citrate. Typically the hydrochloride salt is used.

The amount of tamsulosin active material present in the tablet is relatively low, generally less than 5%, typically 0.1 to 1.5 %. As used herein all percentages refer to weight percent based on the entire weight of the tablet without taking into account any coating thereon, unless otherwise indicated. Typically the amount of tamsulosin active material is within the range of 0.1 to 1.2 %, more typically 0.2 to 1.0 %, preferably 0.2 to 0.8 %, and in many embodiments 0.3 to 0.6 %. In absolute terms, the amount of tamsulosin active material is within the range of 0.1 to 10 mg, generally within the range of 0.1 to 1.2 mg, typically 0.3 to 1.2 mg, and preferably 0.3 to 0.8 mg, expressed in terms of the amount of free base. For example, 0.4 mg of tamsulosin HCl is a preferred amount of tamsulosin which corresponds to 0.367 mg of tamsulosin free base. A preferred embodiment of the present invention contains 0.4 mg +/- 0.04 of tamsulosin HCl or multiples thereof; i.e., 0.2 or 0.8 mg of tamsulosin HCl.

The tablets of the present invention further contain a polymeric matrix. Specific examples of suitable polymeric materials include water swellable cellulosic derivatives such as hydroxypropylmethyl cellulose (HPMC), carboxymethyl cellulose, cellulose acetate, hydroxyethyl cellulose, hydroxypropyl cellulose; sodium alginate; acrylates, methacrylates and co-polymers thereof with various co-monomers; and polyvinyl pyrrolidones.

In particular, it has been surprisingly found that while acrylates and methacrylates such as Eudragits® (Rohm) as well as celluloses can provide pH independent release, the celluloses generally provide for better food effect properties. Hydroxyethyl cellulose, hydroxpropyl cellulose, cellulose acetate, sodium alginate, carboxymethyl cellulose and hydroxypropyl methylcellulose (HPMC) are preferred, with HPMC being the most preferred. The amount of HPMC is generally within the range of 10 to 90 %, typically 20 to 60%, preferably 25 to 40 %, more preferably 30 to 40%, still more preferably 30 to 35 %, based on the total weight of the tablet.

The tablets typically contain additional pharmaceutically acceptable excipients, such as diluents, binders, lubricants, glidants, colorants, preservatives, pH-adjusters, etc. The excipients are selected based on the desired physical aspects of the final form, the desired release rate of the active substance from the composition after its ingestion, and the ease/cost of manufacture. In general the tablets of the present invention contain in addition to the polymeric matrix, at least one carbohydrate and/or compressible diluent. Carbohydrates include lactose, mannitol, maltodextrin, cyclodextrins, dextrates, and dextrin. Compressible diluents include any pharmaceutically acceptable diluent that is suitable for direct compression especially calcium phosphates such as calcium hydrogen phosphate dihydrate and anhydrate forms.

In a preferred embodiment of the present invention, the tablet comprises HPMC and a calcium phosphate such as dicalcium phosphate anhydrate. This embodiment preferably additionally contains a carbohydrate such as lactose anhydrate. A lubricant such as magnesium stearate is also preferably included. The relative amounts are not particularly limited, but it is preferred that these two or three excipients (cellulosic polymer, diluents, and lubricant) comprise the majority of the excipients, such as 95% or more. For example, tablets can comprise 25 to 45% HPMC, 0-50% calcium phosphate (or other insoluble diluent), and 0 to 50 % lactose (or other soluble diluent). The following amounts are preferred: 25 to 40% HPMC, 25-40% calcium phosphate, and 25 to 40 % lactose. More preferably the tablet contains about 30 to 40 % HPMC. Substantially equal amounts of HPMC, calcium phosphate and lactose, i.e. each around 30 to 35 % for a total of 90 to 99.9% is a particularly preferred embodiment. Additional excipients, including a lubricant, etc. may also be present. This preferred tablet formulation generally exhibits the preferred dissolution profiles described-above.

The tablet of the invention is preferably a monolithic tablet, i.e. a tablet which does not disintegrate after ingestion to form a plurality of smaller particles from which the active component is finally released. Instead, the product erodes in the body and/or drug diffuses through the polymer gel releasing the active substance. Thus, in a monolithic tablet embodiment, none of the excipients used in the manufacturing process of the invention should serve as a disintegrant.

Any of the tablets may be coated such as with an enteric coat or simply for color or stability reasons. For therapeutic purposes, bioabsorption of tamsulosin in body fluids should preferably proceed in the small intestines. Accordingly, the tablets of the invention may also be protected by a suitable gastro-resistant coating which delays the onset of release of the active component from the tablet matrix during the passage thereof in the stomach, but this is not necessary to obtain the desired profiles. Examples of such suitable material for gastro-resistant coating are cellulose acetate phthalate (CAP) (Aquacoat CPD™), co-processed polyvinyl acetate phthalate (PVAP) (Suretetic™), cellulose acetate trimellitate (CAT), Eudragit-type polymers (acrylic - methacrylic acid copolymers), hydropropyl methyl cellulose acetate succinate (HPMCAS).

The release property of the coating may be tested also by the same dissolution tests of the uncoated tablets. The preferred properties of a coated tablet are, e.g.:
- Dissolution of the dosage form in SGF, a maximum of 20 % of tamsulosin is released in 2 hours.
- In the other media, the coated tablets should comply with the same dissolution profile as specified above.

The tablets of the present invention can be used directly as a unit dosage form, with or without coating, or two or more tablets can be combined such as in a capsule to form a unit dose. The unit dosage form contains an effective amount of tamsulosin for treating or ameliorating the disease, symptoms, and/or or conditions associated with BPH, hypertension, or congestive heart failure, generally from 0.01 to 10.0 mg, preferably 0.1 to 1 mg, in terms of the free base. Preferable are unit doses comprising 0.2, 0.4 or 0.8 mg tamsulosin hydrochloride per se. A unit dose is normally taken from 1 to 3 times daily, preferably once a day as mentioned above. In the case of a capsule, a sufficient number of tablets are provided based on the concentration of the tamsulosin active material therein, so as to provide an effective amount.

Taking the usual therapeutic dose of tamsulosin into consideration, a tablet with a total mass of not more than 400 mg and normally between 10 and 300 mg are preferred. As the therapeutic dose of tamsulosin is relatively low, the overall weight of the tablet is advantageously kept as low as possible. Low overall weight of a tablet increases the relative content of tamsulosin in the tablet and thus improves the content uniformity. Furthermore, a small tablet will have a similar rate of the gastro-intestinal transit as the granulated product; thus, results obtained from in-vitro dissolution tests may better predict the actual bioequivalence with the marketed multiparticulate product. From this aspect, preferred tablet weight within the invention is from 25 to 250 mg, more preferably 40 to 200 mg, though it is not limited to this range. The most preferred tablet weight is within the range of 80 to 100, especially approximately 100 mg.

Accordingly, the tablets of the invention may be either small, whereby - whenever produced in a circular shape- the average diameter thereof is from about 1.5 to about 2.5 mm, or they may be produced as normal tablets, with an average diameter of between 2.5 and 15 mm, more usually 2.5 to 10 mm. Apart from a circular shape, tamsulosin compositions may be compressed into oval, round biconvex, pentagonal circumcircle or other suitable tablet shape.

Tablets of the invention comprising unit dosage amount of tamsulosin may be delivered for immediate use in a suitable package unit comprising advantageously from 5 to 100 tablets. Such package may comprise a blister pack comprising advantageously 10, 14, 20, 28 or 30 tablets, or a plastic or glass container/bottle containing the same amount (5 to 100) of tablets. Any suitable pharmaceutically acceptable package material may be used in production the package unit.

The tablets of the present invention can be made by any suitable process of tabletting. For example, the tablets can be made by wet granulation wherein the granules are first formed and then, optionally with the addition of further excipient(s) compressed into a tablet. Alternatively, the tablets can be made by dry processes such as direct compression or dry granulation, the latter sometimes being referred to as dry compaction. Preferably the tablets are made by a dry technique in view of manufacturing ease and economy. Because of the small amount of tamsulosin present in the tablet, it is preferred that multiple blending and/or milling steps be carried out in any dry process.

Tablets for oral administration of tamsulosin according to the invention may be used in the management of functional treatment of symptomatic benign prostate hypertrophy or hyperplasia (BPH) or other disorders treatable by tamsulosin (the Disorders). Accordingly, the present invention further provides a method for treating the symptoms of benign prostatic hyperplasia which comprises administering to a patient in need thereof one or more of any of the above-described tablets. The tablets can be administered in a single capsule.

Tablet compositions of the invention may be also used in medical applications in combination with other agents. The combination may be realized in a form of single combination preparation or by separate administration of drugs containing the above agents.

The invention is further illustrated by the following non-limiting Examples.

### Reference Example

Commercially available tamsulosin hydrochloride capsules were obtained in Europe and subjected to dissolution testing, the average of six trials, in each of SGF, SIF, FaSSIF, and FeSSIF in 500 ml of each media at paddle speed 100 rpm in USP apparatus 2 at 37 °C. The amount of drug released was determined by an HPLC method using an HPLC Agilent 1100 system. The detection was made with UV at 230 nm. The results, shown in figure 1, indicate that at 2 hours elapsed time: less than 20% tamsulosin is released in SGF, more than 60% tamsulosin is released in SIF, more than 75% tamsulosin is released in FaSSIF, and less than 40% tamsulosin is released in FeSSIF.

### Example 1

Three batches of monolithic tablets were made by progressive mixing and direct compression with the following characteristics:

### a) Tablet composition

| | | (%) |
|---|---|---|
| Tamsulosin hydrochloride | 0.4 mg | 0.5 |
| Lactose anhydrous | 26.4 mg | 33.0 |
| Dicalcium phosphate anh. | 26.4 mg | 33.0 |
| Hypromelose (HPMC) | 26.4 mg | 33.0 |
| Magnesium stearate | 0.4 mg | 0.5 |
| Total | 80 mg | 100 |

The difference between batches was only in the viscosity value of hypromelose selected:
Batch A contained METHOCEL K4M CR PREMIUM
Batch B contained METHOCEL K15M CR PREMIUM
Batch C contained METHOCEL K100M CR PREMIUM

### b) Modus operandi

Tamsulosin hydrochloride was blended (15 min) with anhydrous lactose in a 1:9 ratio (10% of active substance), milled (15 seconds) and blended again (5 min). This preblend was then mixed with the rest of the lactose, dicalcium phosphate and hypromelose (10 min), and finally magnesium stearate was added and mixed (5 min) to form the precompression blend. This progressive mixing system provided tamsulosin homogeneity in the preblend of 97.2-100.4% and in the precompression blend of 88.1-98.6%). Compression was performed in a Korsch EK0 press at standard speed and pressure.

### c) Characterization of the produced tablets

| Batch | Weight (mg) | Hardness (N) | Height (mm) | Diameter (mm) | Asay (%) |
|---|---|---|---|---|---|
| A | 82.8 | 52 | 2.63 | 5.98 | 93.6 |
| B | 83.5 | 38 | 2.69 | 5.99 | 98.6 |
| C | 81.8 | 52 | 2.66 | 5.99 | 98.6 |

### d) Dissolution studies

Dissolution tests were performed using standard USP apparatus 2, paddles at 50 rpm in 500 ml of SIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm. Results comply with the following specification:
<40 % in 30 minutes
20-60 % in 2 hours
>75 % in 6 hours

### Example 2

Three batches of monolithic tablets were made by progressive mixing and direct compression with the following characteristics:

### a) Tablet composition

| | D | E | F |
|---|---|---|---|
| Tamsulosin hydrochloride | 0.4 mg | 0.4 mg | 0.4 mg |
| Lactose anhydrous | 35.2 mg | 30.8 mg | 22.0 mg |
| Diclcium phosphate anh. | 35.2 mg | 17.6 mg | 35.2 mg |
| Hypromelose (HPMC) | 8.8 mg | 17.6 mg | 35.2 mg |
| Magnesium stearate | 0.4 mg | 0.4 mg | 0.4 mg |
| Total | 80 mg | 80 mg | 80 mg |

The difference between batches was only in the concentration of hypromelose used:
Batch D contained 11% METHOCEL K100M CR PREMIUM
Batch E contained 22 % METHOCEL K100M CR PREMIUM
Batch F contained 44 % METHOCEL K100M CR PREMIUM

### b) Modus operandi

Tamsulosin hydrochloride was blended (15 min) with anhydrous lactose in a 1:9 ratio (10% of active substance), milled (15 seconds) and blended again (5 min). This preblend was then mixed with the rest of the lactose, dicalcium phosphate and hypromelose (10 min), and finally magnesium stearate was added and mixed (5 min). Compression was performed in a Korsch EK0 press.

### c) Characterization of the produced tablets

| Batch | Weight (mg) | Hardness (N) | Height (mm) | Diameter (mm) | Assay (%) |
|---|---|---|---|---|---|
| D | 80.5 | 19 | 2.65 | 6.0 | 91.9 |
| E | 80.4 | 22 | 2.74 | 6.0 | 94.1 |
| F | 80.3 | 22 | 2.97 | 6.0 | 90.5 |

### d) Dissolution studies

Dissolution tests were performed using USP apparatus 1, baskets at 100 rpm and USP apparatus 2, paddles, at 50 rpm, both in 500 ml of SIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm.
Results vary according to HPMC concentration, and in each condition, there is a wide range of curves complying with the intended specification. These results can be extrapolated to other bio-relevant dissolution conditions.

### Example 3

Two batches of monolithic tablets were made by progressive mixing and direct compression with the following characteristics:

### a) Tablet composition

| | | (%) |
|---|---|---|
| Tamsulosin hydrochloride | 0.4 mg | 0.5 |
| Lactose anhydrous | 25.6 mg | 32.0 |

| | | |
|---|---|---|
| Dicalcium phosphate anh. | 25.6 mg | 32.0 |
| Hypromelose (HPMC) | 28.0 mg | 35.0 |
| Magnesium stearate | 0.4 mg | 0.5 |
| Total | 80 mg | 100 |

The differences between both batches were mainly scaling-up, mixing times and physical parameters.
Batch G scaled-up to 20000 units
Batch H scaled-up to 40000 units

### b) Modus operandi

Tamsulosin hydrochloride was blended (Turbula; 15 min) with anhydrous lactose in a 1:9 ratio (10 % of active substance), milled (IKA; 30 seconds) and blended again (Turbula; 5 min). This pre-blend was then mixed with the rest of the lactose, dicalcium phosphate and hypromelose (Bohle LM40). Three progressive mixing times (15, 30 and 45 minutes) were evaluated for batch D and homogeneity was excellent in all cases (tamsulosin assay of 101.2 %, 101.7 % and 102.1 %). Batch E was mixed for only 10 minutes and acceptable homogeneity was also reached. Sieving of dry blends and excipients was done as required to obtain homogeneity. Finally magnesium stearate was sieved, added and mixed (Bohle LM40; 5 min). The precompression blends were compressed in either an eccentric press Korsch EK0 or in a rotary press Korsch XL100 (about 15000-30000 tablets). Compression performed in Korsch XL100 instrumented rotary press was done at high speeds with standard precompression and pressure. Tablet hardness in both batches was changed to study dissolution performance.

### c) Characterization of the produced tablets

| Batch | Weight (mg) | Hardness (N) | Height (mm) | Diameter (mm) | Assay (%) |
|---|---|---|---|---|---|
| G | 80.9 | 85 | 2.55 | 6.00 | 103.2 |
| H | 76.4 | 39 | 2.38 | 5.97 | 98.2 |

### d) Dissolution studies

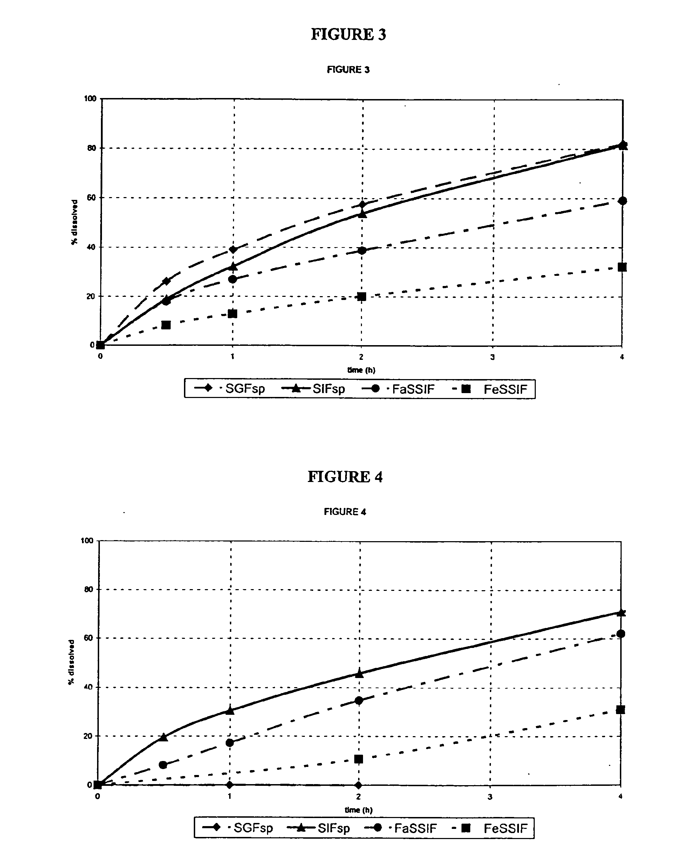

Dissolution tests were performed using standard USP apparatus 2, paddles at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm. The corresponding curves are given in Figures 2 (G) and 3 (H). Results in SGF and SIF comply with the following specification:
<40 % in 30 minutes
20-60 % in 2 hours
>75 % in 6 hours

### e) coating

These tablets were then coated with an enteric polymer (polymethacrylate type C) based on Eudragit L30D55, with additives including triethylcitrate and talc, or with Acryl-Eze ® (available from Colorcon).

### f) Dissolution studies

Dissolution tests of the coated batches were performed using standard USP apparatus 2, paddles at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm.
The corresponding curves are given in Figures 4 (coated G) and 5 (coated H). Results in SIF comply with the following specification:
<40 % in 30 minutes
20-60 % in 2 hours
>75 % in 6 hours

### Example 4

Two batches of tablets were manufactured by a process that includes dry compaction, milling, mixing and compression.

### a) Tablet composition

| | I | J |
|---|---|---|
| Tamsulosin hydrochloride | 0.4 mg | 0.4 mg |
| Lactose | 65.6 mg | 215.6 mg |
| Hypromelose (HPMC) | 33.0 mg | 33.0 mg |
| Magnesium stearate | 1.0 mg | 1.0 mg |
| Total | 100 mg | 250 mg |

Batch I: Monolithic tablet, 6 mm diameter. Contains 33.0% of HPMC K15M P
Batch J: Monolithic tablet, 9 mm diameter. Contains 13.2% of HPMC K15M P

### b) Modus operandi

Tamsulosin was blended (15 min), milled (15 seconds) and blended again (5 min), with anhydrous lactose in a 1:9 ratio (10% of active substance). This preblend was then mixed with the rest of lactose, hypromelose and 25% of magnesium stearate (10 min), compacted in Chilsonator (Fitz-Patrick) and milled in Fitz-Mill (Fitz-Patrick), finally the rest of magnesium stearate (75%) was added and then mixed (15 min). Compression was performed in a Korsch EK0 press at standard speed and pressure.

### c) Characterization of the produced tablets.

| Batch | Weight (mg) | Hardness (N) | Height (mm) | Diameter (mm) | Lubrication coefficient |
|---|---|---|---|---|---|
| I | 103.3 | 31 | 3.42 | 6.0 | 98.4 |
| J | 251.6 | 42 | 3.71 | 9.0 | 100 |

### Example 5

Two batches of tablets were manufactured with different active concentration, tablet geometries and total mass.

### a) Tablet composition

| | K | L | M |
|---|---|---|---|
| Tamsulosin hydrochloride | 0.4 mg | 0.2 mg | 0.2 mg |
| Lactose anhydrous | 25.6 mg | 12.9 mg | 129.0 mg |
| Dicalcium phosphate anh. | 25.6 mg | 12.7 mg | 128.8 mg |
| Hypromelose (HPMC) | 28.0 mg | 14.0 mg | 140.0 mg |
| Magnesium stearate | 0.4 mg | 0.2 mg | 2.0 mg |
| Total | 80 mg | 40 mg | 400 mg |

### b) Modus operandi

Micronised tamsulosin was blended progressively with anhydrous lactose. This preblend was then mixed with the rest of lactose and hypromelose and after with magnesium stearate to provide in all cases enough homogeneity. Compression was performed in a Korsch EK0 press at standard speed and pressures.

### c) Characterization of the produced tablets

| Batch | Weight (mg) | Hardness (N) | Height (mm) | Diameter (mm) | Assay (%) |
|---|---|---|---|---|---|
| K (special shape) | 84.1 | 53 | 2.65 | n.a. | 90.5 |
| L | 40.6 | 36 | 2.01 | 5.00 | 94.1 |
| M | 398.1 | 132 | 4.57 | 10.05 | 90.5 |

### Example 6

A batch of monolithic tablets was manufactured to check dissolution specifications.

### a) Tablet composition

| | | (%) |
|---|---|---|
| Tamsulosin hydrochloride | 0.4 mg | 0.5 |
| Lactose anhydrous | 31.6 mg | 39.5 |
| Dicalcium phosphate anh. | 31.6 mg | 39.5 |
| Hypromelose (HPMC) | 16.0 mg | 20.0 |
| Magnesium stearate | 0.4 mg | 0.5 |
| Total | 80 mg | 100 |

Batch N scale-up was performed using micronised drug substance and less mixing steps. 40000 units were manufactured.

### b) Modus operandi

Micronised tamsulosin hydrochloride was blended (Bohle LM40; 15 min) with hypromelose. This pre-blend was then mixed with lactose and dicalcium phosphate (Bohle LM40; 15 minutes). Sieving of dry blends and excipients was done as required to obtain homogeneity. Finally magnesium stearate was sieved, added and mixed, two times (Bohle LM40; 5 and 15 minutes).
The precompression blends were then compressed. Compression was performed in a Korsch XL100 instrumented rotary press was done at high speeds with standard precompression and pressure.
Characterisation of the tablets is presented below:

### c) Characterization of the produced tablets

| Batch | Weight (mg) | Hardness (N) | Height (mm) | Diameter (mm) | Assay (%) |
|---|---|---|---|---|---|
| N | 80.7 | 47 | 2.50 | 6.05 | 104.0 |

### d) coating

This tablets were then coated with an enteric polymer (Acryl-Eze ® available from Colorcon).

### e) Dissolution studies

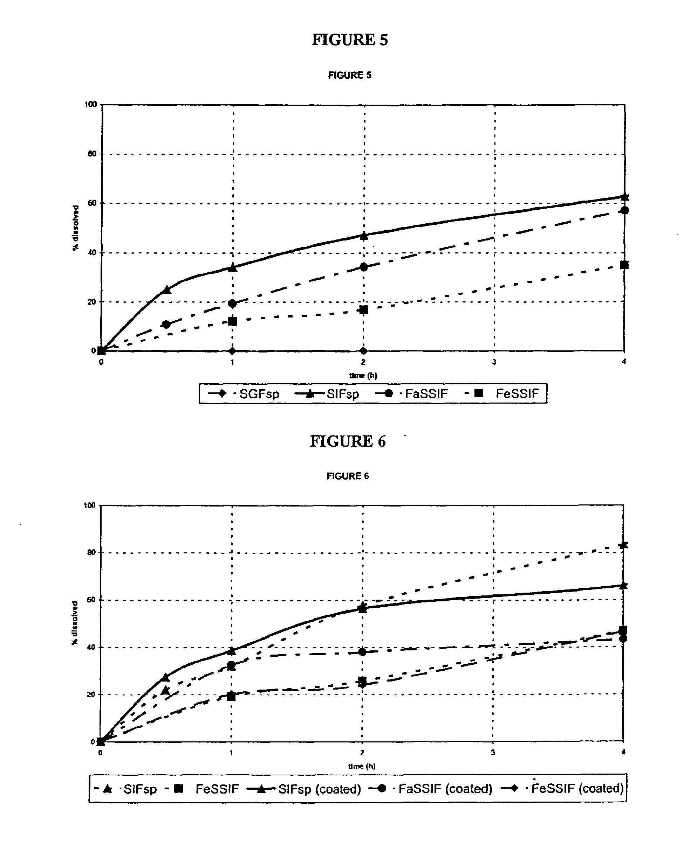

Dissolution tests of the coated batches were performed using standard USP apparatus 2, paddles, at 100 rpm in 500 ml of SGF, SIF, FaSSIF and FeSSIF. The drug released was determined by an HPLC method using an HPLC Agilent 1100 system. The analysis was performed with a guard column and a C₁₈ analytical column, using an isocratic elution mode, with phosphate buffer pH 6.5 and acetonitrile (65:35) as eluents. The detection was made with UV at 230 nm.
The corresponding curves are given in Figure 6.
Results in all media comply with the following specification:
<40 % in 30 minutes
20-60 % in 2 hours
>75 % in 6 hours
The invention is also described in the following clauses:

### Clauses

1. A pharmaceutical tablet comprising a tablet matrix having dispersed therein 0.1 to 10 mg of tamsulosin or a pharmaceutically acceptable salt thereof, and optionally having an enteric coating over said matrix, wherein said tablet is a modified release tablet and has a dissolution profile such that in each of the media SIF, FaSSIF, and FeSSIF, said tablet releases not more than 60% of said tamsulosin at 2 hours elapsed time in USP 2 apparatus using 500 ml of said media at 50-100 rpm paddle speed.
2. The pharmaceutical tablet according to clause 1, wherein said dissolution profile is measured using 100 rpm paddle speed.
3. The pharmaceutical tablet according to clause 1 or 2, wherein said dissolution profile exhibits a release of at least 20% in each of said media at 2 hours elapsed time.
4. The pharmaceutical tablet according to clause 1-3, wherein said tablet has a dissolution profile wherein the amount of tamsulosin released at 2 hours in FeSSIF media is at least 50% the amount released at 2 hours in FaSSIF media.
5. The pharmaceutical tablet according to clause 4 , wherein the amount of tamsulosin released at 2 hours in FeSSIF media is at least 60% the amount released at 2 hours in FaSSIF media.
6. The pharmaceutical tablet according to clause 1-5, wherein said tablet has a dissolution profile wherein said tablet releases not more than 60% of said tamsulosin at 2 hours elapsed time in USP 2 apparatus using 500 ml of SGF media at 100 rpm paddle speed.
7. The pharmaceutical tablet according to clause 1-6, wherein said dissolution profile includes releasing less than 40% of the tamsulosin in 30 minutes, 20 - 60% of the tamsulosin in 2 hours, and greater than 75% of the tamsulosin in 6 hours in USP 2 apparatus using 500 ml of SIF media at 100 rpm paddle speed.
8. The pharmaceutical tablet according to clause 1-7, wherein said dissolution profile includes releasing less than 40% of the tamsulosin in 30 minutes, 20 - 60% of the tamsulosin in 2 hours, and greater than 75% of the tamsulosin in 6 hours in USP 2 apparatus using 500 ml of SGF media at 100 rpm paddle speed.
9. The pharmaceutical tablet according to clause 1-8, wherein said tablet has an enteric coating.
10. The pharmaceutical tablet according to clauses 1-9, wherein the material of the coating comprises at least one compound selected from the group consisting of cellulose acetate phthalate (CAP) (Aquacoat CPD™), co-processed polyvinyl acetate phthalate (PVAP) (Suretetic™), cellulose acetate trimellitate (CAT), Eudragit-type polymers (acrylic -methacrylic acid copolymers), hydropropyl methyl cellulose acetate succinate (HPMCAS).
11. The pharmaceutical tablet according to clause 1-8, wherein said tablet does not have an enteric coating.
12. The pharmaceutical tablet according to clause 11, wherein said tablet is uncoated.
13. The pharmaceutical tablet according to clause 1-12, wherein said tablet matrix comprises a polymeric matrix.
14. The pharmaceutical tablet according to clauses 1-13 wherein the matrix component is selected from the group comprisig a water swellable cellulosic derivative; sodium alginate; acrylates, methacrylates and co-polymers thereof with various co-monomers; and polyvinyl pyrrolidones.
15. The pharmaceutical tablet according to clauses 14 , wherein the water swellable cellulosic derivative is hydroxypropylmethyl cellulose (HPMC), carboxymethyl cellulose, cellulose acetate, hydroxyethyl cellulose, hydroxypropyl cellulose.
16. The pharmaceutical tablet according to clause 15, wherein said tablet matrix comprises hydroxypropyl methylcellulose.
17. The pharmaceutical tablet according to clause 15-16, wherein said tablet comprises said hydroxypropyl methylcellulose in an amount within the range of 10 wt% - 90wt%.
18. The pharmaceutical tablet according to clause 17, wherein said tablet comprises said hydroxypropyl methylcellulose in an amount within the range of 25 wt% - 40wt%.
19. The pharmaceutical tablet according to clause 18, wherein said tablet comprises said hydroxypropyl methylcellulose in an amount within the range of 30 wt% - 35 wt%.
20. The pharmaceutical tablet according to clause 1-19, wherein said tamsulosin or pharmaceutically acceptable salt thereof is contained in an amount of 0.2 to 1.0 %.
21. The pharmaceutical tablet according to clause 20, wherein said tamsulosin or pharmaceutically acceptable salt thereof is contained in an amount of 0.2 to 0.8 %.
22. The pharmaceutical tablet according to clause 1-21, wherein said tamsulosin or pharmaceutically acceptable salt thereof is tamsulosin hydrochloride and said tamsulosin hydrochloride is contained in an amount of 0.4mg +/- 0.04.
23. The pharmaceutical tablet according to clause 1-22, which further comprises at least one pharmaceutically acceptable excipient selected from the group consisting of a carbohydrate and a compressible diluent.
24. The pharmaceutical tablet according to clause 23, which further comprises lactose.
25. The pharmaceutical tablet according to clause 23-24, which comprises a calcium phosphate.
26. The pharmaceutical tablet according to clause 1-25, which comprises lactose, HPMC, a calcium phosphate, and magnesium stearate.
27. The pharmaceutical tablet according to clause 1-26, wherein said tablet is a once daily dose tablet.
28. The pharmaceutical tablet according to clauses 1-27 , which does not comprise a disintegrant.
29. A pharmaceutical tablet comprising a tablet matrix having dispersed therein 0.1 to 10 mg of tamsulosin or a pharmaceutically acceptable salt thereof, and optionally having an enteric coating over said matrix, wherein said tablet is a modified release tablet and exhibits¹, when administered by a human patient in fasted state, a maximum plasma concentration ,which is less than 40% higher than that when administered in fed state.
30. The pharmaceutical tablet according to clause 29, wherein said tablet has an enteric coating.
31. The pharmaceutical tablet according to clause 29-30, wherein the material of the coating comprises at least one compound selected from the group consisting of cellulose acetate phthalate (CAP) (Aquacoat CPD™), co-processed polyvinyl acetate phthalate (PVAP) (Suretetic™), cellulose acetate trimellitate (CAT), Eudragit-type polymers (acrylic -methacrylic acid copolymers), hydropropyl methyl cellulose acetate succinate (HPMCAS).
32. The pharmaceutical tablet according to clause 29, wherein said tablet does not have an enteric coating.
33. The pharmaceutical tablet according to clause 32, wherein said tablet is uncoated.
34. The pharmaceutical tablet according to clauses 29-33, wherein said tablet matrix comprises a polymeric matrix.
35. The pharmaceutical tablet according to clauses 29-35 as in 1-11 wherein the polymeric matrix component is selected from the group comprisig a water swellable cellulosic derivative; sodium alginate; acrylates, methacrylates and copolymers thereof with various co-monomers; and polyvinyl pyrrolidones.
36. The pharmaceutical tablet according to clause 35 , wherein the water swellabble cellulosic derivative is hydroxypropylmethyl cellulose (HPMC), carboxymethyl cellulose, cellulose acetate, hydroxyethyl cellulose, hydroxypropyl cellulose.
37. The pharmaceutical tablet according to clause 36, wherein said tablet matrix comprises hydroxypropyl methylcellulose.
38. The pharmaceutical tablet according to clause 35-36, wherein said tablet comprises said hydroxypropyl methylcellulose in an amount within the range of 10 wt% - 90wt%.
39. The pharmaceutical tablet according to clause 38, wherein said tablet comprises said hydroxypropyl methylcellulose in an amount within the range of 25 wt% - 40wt%.
40. The pharmaceutical tablet according to clause 39, wherein said tablet comprises said hydroxypropyl methylcellulose in an amount within the range of 30 wt% - 35 wt%.
41. The pharmaceutical tablet according to clause 29-40, wherein said tamsulosin or pharmaceutically acceptable salt thereof is contained in an amount of 0.2 to 1.0 %.
42. The pharmaceutical tablet according to clause 41, wherein said tamsulosin or pharmaceutically acceptable salt thereof is contained in an amount of 0.2 to 0.8 %.
43. The pharmaceutical tablet according to clause 29-42, wherein said tamsulosin or pharmaceutically acceptable salt thereof is tamsulosin hydrochloride and said tamsulosin hydrochloride is contained in an amount of 0.4mg +/- 0.04.
44. The pharmaceutical tablet according to clause 29-43, which further comprises at least one pharmaceutically acceptable excipient selected from the group consisting of a carbohydrate and a compressible diluent.
45. The pharmaceutical tablet according to clause 44, which further comprises lactose.
46. The pharmaceutical tablet according to clause 44-45, which comprises a calcium phosphate.
47. The pharmaceutical tablet according to clause 29-46, which comprises lactose, HPMC, a calcium phosphate, and magnesium stearate.
48. The pharmaceutical tablet according to clause 29-47, wherein said tablet is a once daily dose tablet.
49. The pharmaceutical tablet according to clauses 29-48 , which doesnot comprise a disintegrant.
50. A monolithic pharmaceutical tablet, comprising 0.1 to 10 mg of tamsulosin or a pharmaceutically acceptable salt thereof, 10 wt% - 90 wt% hydroxypropyl methylcellulose, and a total tablet weight of 10 to 300 mg.
51. The monolithic tablet according to clause 50, wherein said total tablet weight is within the range of 25 to 250 mg.
52. The monolithic tablet according to clause 51, wherein said total tablet weight is within the range of 80 to 100 mg.
53. The monolithic tablet according to clause 50-52, which comprises said hydroxypropyl methylcellulose in an amount within the range of 25 wt% - 40 wt%.
54. The monolithic tablet according to clause 50-53, which comprises said hydroxypropyl methylcellulose in an amount within the range of 30 wt% - 40 wt%.
55. The monolithic tablet according to clause 50-54, wherein said tablet comprises said hydroxypropyl methylcellulose in an amount within the range of 30 wt% - 35 wt%.
56. The monolithic tablet according to clause 50-55, wherein said tablet further comprises a calcium phosphate, lactose, mannitol, or a combination thereof.
57. The monolithic tablet according to clause 56, wherein said tablet comprises dibasic calcium phosphate anhydrous.
58. The monolithic tablet according to clause 50-57, which does not contain an enteric coating.
59. A unit dosage form for treating or ameliorating the conditions of benign prostatic hyperplasia comprising an effective amount of one or more tablets according to clause 1-58.
60. The unit dosage form according to clause 60, which comprises two or more of said tablets in a capsule.

The invention having been described, it will be readily apparent to those skilled in the art that further changes and modifications in actual implementation of the concepts and embodiments described herein can easily be made or may be learned by practice of the invention, without departing from the spirit and scope of the invention as defined by the following claims.

## Claims

1. A pharmaceutical tablet comprising a tablet matrix having dispersed therein 0.1 to 10 mg of tamsulosin or a pharmaceutically acceptable salt thereof, and optionally having an enteric coating over said matrix, wherein said tablet is a modified release tablet and exhibits', when administered by a human patient in fasted state, a maximum plasma concentration ,which is less than 40% higher than that when administered in fed state.

2. The pharmaceutical tablet according to claim 1, wherein said tablet has an enteric coating.

3. The pharmaceutical tablet according to claim 1 or 2, , wherein the material of the coating comprises at least one compound selected from the group consisting of cellulose acetate phthalate (CAP) (Aquacoat CPD™), co-processed polyvinyl acetate phthalate (PVAP) (Suretetic™), cellulose acetate trimellitate (CAT), Eudragit-type polymers (acrylic - methacrylic acid copolymers), hydropropyl methyl cellulose acetate succinate (HPMCAS).

4. The pharmaceutical tablet according to claim 1, wherein said tablet does not have an enteric coating.

5. The pharmaceutical tablet according to claim 4 wherein said tablet is uncoated.

6. The pharmaceutical tablet according to claims 1-5, wherein said tablet matrix comprises a polymeric matrix.

7. The pharmaceutical tablet according to claims 1 - 6, wherein the polymeric matrix component is selected from the group comprisig a water swellable cellulosic derivative; sodium alginate; acrylates, methacrylates and co-polymers thereof with various co-monomers; and polyvinyl pyrrolidones.

8. The pharmaceutical tablet according to claim 7, wherein the water swellabble cellulosic derivative is hydroxypropylmethyl cellulose (HPMC), carboxymethyl cellulose, cellulose acetate, hydroxyethyl cellulose, hydroxypropyl cellulose.

9. The pharmaceutical tablet according to claim 8 , wherein said tablet matrix comprises hydroxypropyl methylcellulose.

10. The pharmaceutical tablet according to claim 8 or 9, wherein said tablet comprises said hydroxypropyl methylcellulose in an amount within the range of 10 wt% - 90wt%.

11. The pharmaceutical tablet according to claim 10 , wherein said tablet comprises said hydroxypropyl methylcellulose in an amount within the range of 25 wt% - 40wt%.

12. The pharmaceutical tablet according to claim 11, wherein said tablet comprises said hydroxypropyl methylcellulose in an amount within the range of 30 wt% - 35 wt%.

13. The pharmaceutical tablet according to claim 1-12, wherein said tamsulosin or pharmaceutically acceptable salt thereof is contained in an amount of 0.2 to 1.0 %.

14. The pharmaceutical tablet according to claim 13, wherein said tamsulosin or pharmaceutically acceptable salt thereof is contained in an amount of 0.2 to 0.8 %.

15. The pharmaceutical tablet according to claim 1-14, wherein said tamsulosin or pharmaceutically acceptable salt thereof is tamsulosin hydrochloride and said tamsulosin hydrochloride is contained in an amount of 0.4mg +/- 0.04.

16. The pharmaceutical tablet according to claim 1-15, which further comprises at least one pharmaceutically acceptable excipient selected from the group consisting of a carbohydrate and a compressible diluent.

17. The pharmaceutical tablet according to claim 16, which further comprises lactose.

18. The pharmaceutical tablet according to claim 16 or 17, which comprises a calcium phosphate.

19. The pharmaceutical tablet according to claim 1-18, which comprises lactose, HPMC, a calcium phosphate, and magnesium stearate.

20. The pharmaceutical tablet according to claim 1-19, wherein said tablet is a once daily dose tablet.

21. The pharmaceutical tablet according to claims 1-20 , being a monolithic tablet and which does not comprise a disintegrant.
